# EUROPÄISCHE PATENTSCHRIFT

(11) **EP 1 825 765 B1**
(45) Veröffentlichungstag und Bekanntmachung des Hinweises auf die Patenterteilung: **10.04.2019**
(21) Anmeldenummer: 07000218.3
(22) Anmeldetag: 08.01.2007
(51) Int. Cl.: A23N 1/00, C12M 1/00

(54) **Verfahren zum Aufschliessen von biologischem Material sowie Vorrichtung zu seiner Durchführung**
Method for solubilising biological material and device for its application
Procédé destiné à la solubilisation de matière biologique tout comme son dispositif de réalisation

(30) Priorität: 24.02.2006 DE 102006009157
(43) Veröffentlichungstag der Anmeldung: 29.08.2007
(73) Patentinhaber: Eisenmann SE, 71032 Böblingen (DE)
(72) Erfinder: Swoboda, Werner, 71032 Böblingen (DE)
(74) Vertreter: Ostertag & Partner Patentanwälte mbB

(56) Entgegenhaltungen:
- WO-A-2005/025669
- WO-A-2005/056788
- DE-C1- 10 144 486
- US-A- 5 019 034
- US-A1- 2005 048 651
- US-A1- 2005 170 510
- US-B1- 6 617 154

## Beschreibung

Wirkstoffe, die als Heilmittel, Nahrungsergänzungsmittel, Aromen, Duftstoffe, Farben usw. dienen, oder sonstige für die Lebenshaltung oder in der Industrie verwertbare Stoffe, die in der Natur vorkommen, finden sich oft in den Zellen biologischer Materialien. Diese biologischen Materialien sind aus Zellen aufgebaut, die jeweils eine Zellmembran umfassen, welche einen Zellinhalt umschließt.

Um an die verschiedenen Wirkstoffe, die im Zellinhalt, aber auch in der Zellmembran selbst enthalten sind, heranzukommen, muß die Zellmembran geöffnet werden. Hierfür finden insbesondere mechanische Verfahren zum Zerstören der Zellmembran Verwendung. Auch physikalische Verfahren (z.B. Kochen), chemische Verfahren und biologische Verfahren (Fermentierung u. ä.) sind zum Freisetzen von Wirkstoffen bekannt.

Unter Wirkstoff soll hier nicht nur ein Wirkstoff im engen pharmakologischen Sinne verstanden werden, sondern auch jeder wirtschaftlich (durch Anwesenheit oder Abwesenheit) interessante Stoff.

In jüngerer Zeit verwendet man auch die Elektroporation zum Öffnen von Zellmembranen. Die Elektroporation ist jedoch auf solche biologische Materialien beschränkt, die wasser- und/oder ölhaltige Zellen umfassen. Verschiedene Elektroporationsverfahren sind aus den Dokumenten WO 2005/056788 A, US 5 019 034 oder DE 101 44 486 C1 bekannt. Durch die Elektroporation wird verglichen mit den weiter oben angesprochenen bekannten Zellaufschlussverfahren ein schonenderes und wirkungsvolleres Aufschließen erreicht.

In Elektroporationsreaktoren treten zuweilen bei makroskopisch gleicher zu behandelnder Mischung Durchschläge auf, bei denen der gesamte oder ein großer Teil der angelegten Hochspannung über einen dünnen Entladungskanal abgebaut wird. Entsprechend bleibt ein großer Teil des Materiales durch diesen Hochspannungsimpuls unbehandelt. Der durch den Durchschlag geschaffene Entladungskanal oder Teile desselben und dort verbleibende Entladungsprodukte können ferner Keime für weitere Durchschläge bilden.

Auch Änderungen in der Leitfähigkeit des zu behandelnden Materiales können die Wirksamkeit der Elektroporation beeinträchtigen.

Die vorliegende Erfindung hat sich zur Aufgabe gestellt, den Aufschluss der Zellen nochmals zu verbessern, indem den oben genannten Störeinflüssen begegnet wird.

Diese Aufgabe ist erfindungsgemäß gelöst durch ein Verfahren mit den im Anspruch 1 angegebenen Merkmalen.

Den erfindungsgemäßen Verfahren liegt der Gedanke zugrunde, dass es günstig ist, die Elektroporation unter Arbeitsparametern zu fahren, die eine gleichbleibende gute Wirksamkeit der durch die Feldimpulse erzeugten Poration gewährleisten.

Das erfindungsgemäße Verfahren gemäß Anspruch 1 erlaubt auf einfache und eindeutige Weise festzustellen, wann die für die Elektroporation verwendete Feldstärke zu groß ist, bzw. das behandelte Material nicht ausreichend durchschlagfeste Inhomogenitäten enthält.

In der Praxis ist es oft so, dass die Zusammensetzung der Mischung aus Trägermedium und biologischem Material schwankt. So kann es zum Beispiel beim Aufschluss von Trauben Chargen geben, die unterschiedlichen Reifungsgrad und unterschiedlichen Zuckergehalt aufweisen. Je nach dem, wie stark die Reifung fortgeschritten ist, weisen die Zellwände unterschiedliche mechanische Stabilität auf. Entsprechend ist es zweckmäßig, die Höhe der verwendeten Feldimpulse anzupassen. Andererseits kann es bei zu hohen Feldimpulsen zu Durchschlägen im Elektroporationsreaktor kommen, was zur Folge hat, dass der zu einem Durchschlag führende Feldimpuls nicht zur Elektroporation der Zellen beiträgt.

Vorteilhafte Weiterbildungen der erfindungsgemäßen Verfahren sind in Unteransprüchen angegeben.

Gemäß Anspruch 2 kann man die Durchschläge auf einfache Weise mit auf Licht ansprechenden Detektoren feststellen.

Beim Verfahren nach Anspruch 3 werden die Durchschläge anhand von durch die erzeugten Druckstößen in der Mischung detektiert.

Kommt es in der elektroporierten Mischung zu Durchschlägen, so kann man diese auch an der Amplitude und dem zeitlichen Verlauf der Stromimpulse feststellen. Die typischerweise in einem Kondensator gespeicherte Energie wird bei Vorliegen eines Durchschlages sehr viel rascher abgebaut. Hiervon kann man bei dem Verfahren gemäß Anspruch 4 Gebrauch machen.

Auf besonders einfache Weise kann man die Durchschläge dadurch detektieren, dass man die Breite des Stromimpulses durch den Elektroporationsreaktor bzw. des an der Mischung abfallenden Hochspannungsimpulses überwacht, wie im Anspruch 5 angegeben.

Das Verfahren gemäß Anspruch 6 gestattet es, die Leitfähigkeit dauernd zu überwachen, also auch schon in Behandlungsphasen, in denen keine Überschläge auftreten. Auf diese Weise kann man auch solche Fälle feststellen und detektieren, bei denen die elektrische Leitfähigkeit zu klein ist und keine Überschläge auftreten können. Durch Erhöhen der Leitfähigkeit der Mischung kann man dann wieder die für die Elektroporation günstigsten Verhältnisse einstellen. Auch gestattet es diese Weiterbildung, sich erst anbahnenden Ausreißern der Leitfähigkeit schon zu begegnen.

Bei dem Verfahren gemäß Anspruch 7 wird ein Abweichen der Leitfähigkeit der Mischung vom gewünschten Sollwert schon vor der Elektroporation festgestellt. Dies erlaubt eine besonders rasche Korrektur bei einer Vorbehandlung bzw. beim Mischen des biologischen Materiales mit dem Trägermedium.

Der Anspruch 8 gibt verschiedene Parameter für die Elektroporation an, die zunächst geändert werden können, um bei abweichender Leitfähigkeit wieder ein störungsfreies Arbeiten der Elektroporation zu gewährleisten, bis dann nach dem nur langsamer realisierbaren Wiederheranführen der Leitfähigkeit an ihren Sollwert auch die direkten Arbeitsparameter der Elektroporation wieder auf ihre Normalwerte zurückgestellt werden können.

Auch die im Anspruch 9 angegebenen Parameter erlauben es, die Elektroporationsverhältnisse mit verglichen mit der Korrektur der Leitfähigkeit kleiner Zeitkonstante wieder an solche Verhältnisse heranzuführen, in denen die Elektroporation störungsfrei läuft.

Die Ansprüche 10 und 11 betreffen Maßnahmen, die das Wiederheranführen der Leitfähigkeit der Mischung an ihren Sollwert ermöglichen.

Dabei nennt der Anspruch 12 speziell solche Parameter, die bei einer Vorbehandlung von keine oder nur wenig Flüssigkeit enthaltendem biologischem Material vorteilhaft sind. Bei derartigem Material muss in einer Vorbehandlung Flüssigkeit ins Innere der Zellen eingeschleust werden, um dort wieder eine schwache Leitfähigkeit herzustellen.

Auch der Anspruch 13 betrifft Maßnahmen, die das Ändern der Leitfähigkeit der Mischung aus Trägermedium und biologischem Material im Rahmen einer Vorbehandlung beinhalten.

Auch durch Änderung der Rückführungsrate von Material von einer Verfahrensstufe zu einer vorhergehenden Verfahrensstufe gemäß Anspruch 14 kann man die Leitfähigkeit der Mischung steuern.

Soweit dabei der rückgeführte Materialstrom einer Zwischenbehandlung unterworfen wird, kann man auch diese gemäß Anspruch 15 dazu verwenden, die Leitfähigkeit der dem Elektroporationsreaktor zugeführten Mischung einzustellen.

Die Ansprüche 16 und 17 geben Maßnahmen an, wie man eine kurzfristig verminderte oder erhöhte Effektivität der Elektroporation ausgleichen kann und ein gleichbleibendes Ergebnis im elektroporierten Gut gewährleisten kann.

In manchen Fällen kann es notwendig sein, dann, wenn eine schwerere Störung der elektrischen Leitfähigkeit auftritt, was sich an einer Häufung von Durchschlägen erkennen lässt, eine Notmaßnahme zu ergreifen, um die Anlage und/oder das behandelte Material zu schützen. Ein entsprechendes Verfahren ist im Anspruch 18 angegeben. Es versteht sich, daß das Einleiten einer Notmaßnahme auch schon bei einem ersten auftretenden Durchschlag erfolgen kann.

Anspruch 19 gibt bevorzugte Notmaßnahmen an.

In Fällen, in denen man die Leitfähigkeit der Mischung durch Zugabe einer Hilfskomponente (mit)steuert, lässt sich gemäß Anspruch 20 ein rasches Wiederheranführen der Leitfähigkeit an ihren Sollwert bewerkstelligen, bevor dann anschließend über eine Änderung der Vorbehandlung oder über eine andere Mischung des zu behandelnden biologischen Materials mit Trägermedium dafür Sorge getragen wird, dass auch ohne Leitfähigkeitsadditiv oder mit der normalen Menge desselben die gewünschte Leitfähigkeit der Mischung erhalten wird.

Anspruch 21 betrifft eine Vorrichtung zur Durchführung des erfindungsgemäßen Verfahren, die sich unter Verwendung handelsüblicher Sensoren und Schaltkreise realisieren lässt.

Die Vorrichtung gemäß Anspruch 22 liefert laufend ein für die aktuelle Leitfähigkeit der Mischung charakteristisches Signal.

Bevor Ausführungsbeispiele der Erfindung detailliert beschrieben werden, sollen zunächst allgemeine Gesichtspunkte bei der Realisierung der Erfindung behandelt werden, wobei auf den komplexeren Fall des Aufschlusses von flüssigkeitsarmem biologischem Material Bezug genommen wird. Der einfachere Fall des Aufschlusses von biologischem Material, dessen Zellen schon ausreichend Flüssigkeit erhalten, erfolgt sinngemäß, indem man den Schritt der Flüssigkeitsanreicherung weglässt.

Manche biologische Materialien, die Wirkstoffe enthalten, enthalten in ihren Zellen kaum Wasser oder kein Wasser mehr. Es sind dies z.B. getrocknete Pflanzenteile oder Pflanzenteile, die von Hause aus nur geringen Wassergehalt haben, z. B. Rinden, bestimmte Pflanzenwurzeln oder Nadeln von Tannen, Latschen etc..

Erfindungsgemäß wird dem flüssigkeitsarmen biologischen Material zunächst in einem ersten Verfahrensschritt ein Penetrationsmedium zugeführt, das sich in die Zellen einbaut, und in einem zweiten Verfahrensschritt werden die Zellwände des mit Penetrationsmedium angereicherten biologischen Materiales in einem Trägermedium verteilt und durch Elektroporation zerstört.

Es kommen insbesondere flüssige und gasförmige Penetrationsmedien in Betracht. Sie haben generell die Aufgabe die elektrische Leitfähigkeit der Zellen auf einen Wert einzustellen, der im Leitfähigkeitsspektrum flüssigkeitsreicher lebender Zellen liegt, z.B. dem der Blätter von Laubbäumen, Salaten und Früchten. In der Praxis bedeutet dies eine Leitfähigkeit einer aus den letztgenannten Materialien hergestellten Maische von etwa 0,1 mS/cm bis etwa 100 mS/cm, meistens zwischen etwa 0,5 mS/cm und 30 mS/cm.

Unter biologischem Material soll in den Ansprüchen und in der vorliegenden Beschreibung jegliches Material verstanden werden, bei welchem ein Zellinhalt von einer Zellwand umschlossen ist. Es kann sich hierbei sowohl um eukaryotische Materialien (wie pflanzliche Materialien) als auch um prokaryotische Zellen handeln.

Diese Materialien unterscheiden sich nicht unerheblich in der Größe ihrer Zellen. Bei kleinerem Zelldurchmesser wird eine entsprechend höhere elektrische Feldstärke bei der Elektroporation verwendet, um einen Spannungsabfall über den einzelnen Zellen im Bereich von etwa 0,5 V bis etwa 2 V zu erzielen.

Zum Herstellen bzw. Wiederherstellen einer Leitfähigkeit in biologischen Zellen verwendbare Medien werden in den Ansprüchen und in der Beschreibung als Penetrationsmedien bezeichnet, da sie durch die Zellwände oder fehlerhafte Stellen derselben ins Zellinnere eindringen.

Es handelt sich hierbei in erster Linie um Flüssigkeiten und Gase (insbesondere Dämpfe) mit entsprechenden elektrischen Eigenschaften.

Da viele wenig oder kein Wasser enthaltende Zellen Salze enthalten, reicht es oft aus, normales Wasser oder Wasserdampf in die Zellen einzubringen, um dort eine elektrische Leitfähigkeit herbeizuführen.

Unter den geeigneten Penetrationsflüssigkeiten befindet sich nicht nur Wasser, sondern auch wässrige Lösungen, z.B. isotonische Salzlösungen oder vergleichbare biologisch aktive Lösungen. Es können aber auch andere, nicht normalerweise in Zellen angefundene Flüssigkeiten und Gase verwendet werden, die ähnliche elektrische Leitfähigkeit aufweisen, wenn sie nur in einem Flüssigkeits-Vorbehandlungschritt in die Zellen des biologischen Materials aufgenommen werden.

Bei dem erfindungsgemäßen Verfahren werden die mit Penetrationsmedium angereicherten Zellen des biologischen Materiales in einem Trägermedium in ein elektrisches Wechselfeld gebracht, wie es typischerweise für die Elektroporation verwendet wird.

Geeignete Trägermedien haben eine schwache elektrische Leitfähigkeit. Hierunter soll eine Leitfähigkeit verstanden werden, die nicht wesentlich besser, vorzugsweise schlechter ist als die Leitfähigkeit der Zellen, damit die beim Elektroporieren fließenden Stromimpulse nicht an den Zellen vorbeilaufen.

Typische Leitfähigkeitswerte für gute Trägermedien liegen daher im Bereich von wenigen mS/cm.

Eine weitere Grundeigenschaft der Trägermedien ist ihre Durchschlagfestigkeit unter den zum Elektroporieren verwendeten elektrischen Feldern (typischerweise 1 kV/cm bis hin zu 100 kV/cm).

Ein Elektroporationsfeld umfasst eine Vielzahl aufeinanderfolgender schmaler und hoher Feldimpulse, wobei die Feldstärke so hoch gewählt ist, daß schon existierende kleine Poren und/oder Ionenkanäle in den Zellwänden vergrößert werden oder neue Poren in den Zellwänden geschaffen werden.

Als Trägerflüssigkeit eignet sich insbesondere schwach leitendes Wasser. Unter schwach leitend soll in diesem Zusammenhang ein Wasser verstanden werden, welches typische Trinkwasserqualität oder gehobene Brauchwasserqualität aufweist, also ein Wasser mit einer Leitfähigkeit von 0,1mS/cm bis etwa 10 mS/cm.

Anstelle von Wasser können jedoch auch andere Flüssigkeiten verwendet werden, sofern sie die entsprechenden elektrischen Eigenschaften aufweisen, die sicherstellen, daß an den einzelnen Zellen des aufzuschließenden Materiales in gegenüberliegenden Randbereichen Potentialunterschiede von größenordnungsmäßig 0,5 V bis 2 V oder mehr erreicht werden.

Das Aufschließen von biologischem Material kann nicht nur mit der Zielsetzung erfolgen, anschließend an das Aufschließen einen nützlichen Wirkstoff zu extrahieren, sondern auch im Hinblick auf die Zielsetzung, aus dem aufgeschlossenen Material einen unerwünschten Schadstoff zu eliminieren, z.B. in einem Nahrungsmittel einen unerwünschten Bitterstoff oder dergleichen.

Die erfindungsgemäß verwendete Elektroporation ist zwar an sich in Verbindung mit wasserhaltigen biologischen Materialien bekannt, wie der Aufsatz von Bluhm, Frey et al. in den Nachrichten des Forschungszentrums Karlsruhe, Jahrgang 35, Heft 3, 2003, Seiten 105 bis 110 zeigt. Die DE 101 44 486 C1 offenbart auch das Aufschließen und Pasteurisieren organischen Materiales durch Elektroporation am Beispiel von Zuckerrüben.

Diese bekannten Verfahren eignen sich aber, wie eingangs dargelegt, nicht für solche biologische Materialien, die flüssigkeitsarm sind.

Auch sind dort keine Maßnahmen getroffen, die dem Auftreten von Durchschlägen entgegenwirken.

Durch die vorliegende Erfindung wird es dagegen möglich, auch bei heiklen Materialien ein schonendes und sehr effektives Aufschließen der Zellen zu erhalten.

Dies gelingt bei (verglichen mit thermischen Methoden) geringem Energieeinsatz und geringer Erwärmung des biologischen Materiales und damit schonend. Die Ausbeute beim Extrahieren von Wirkstoffen und Schadstoffen in einem sich an das Aufschließen anschließenden Extraktionsschritt ist verbessert. Die Behandlungszeit ist verkürzt. Auch erfolgt der Aufschluß im gesamten Volumen im wesentlichen synchron und damit homogen.

Nachstehend wird die Erfindung anhand von Ausführungsbeispielen unter Bezugnahme auf die Zeichnung näher erläutert. In dieser zeigen:
- Figur 1:: eine schematische Darstellung eines erfindungsgemäßen Verfahrens zum Aufschließen von getrocknetem oder wasserarmem Pflanzenmaterial;
- Figur 2:: eine schematische Darstellung einer Anlage zum Einbringen von Flüssigkeit in getrocknetes biologisches Material sowie zur Elektroporation des wieder mit Flüssigkeit angereicherten biologischen Materiales;
- Figur 3:: eine ähnliche Darstellung wie Figur 1, in welcher jedoch ein abgewandeltes Verfahren wiedergegeben ist;
- Figur 4:: eine ähnliche Darstellung wie Figur 2, wobei jedoch der Elektroporationsreaktor vertikal ausgerichtet ist und eine abgewandelte Hochspannungs-Schalteinrichtung Verwendung findet;
- Figur 5:: eine ähnliche Ansicht wie Figur 4, bei welcher jedoch eine nochmals abgewandelte Hochspannungsschalteinrichtung gezeigt ist;
- Figur 6:: ein Blockschaltbild einer Hauptroutine, nach welcher ein Rechner die Leitfähigkeit der durch den Elektroporationsreaktor bewegten Mischung aus Trägermedium und biologischem Material einstellt; und
- Figur 7:: eine Unterroutine zu der in Figur 6 gezeigten Hauptroutine.

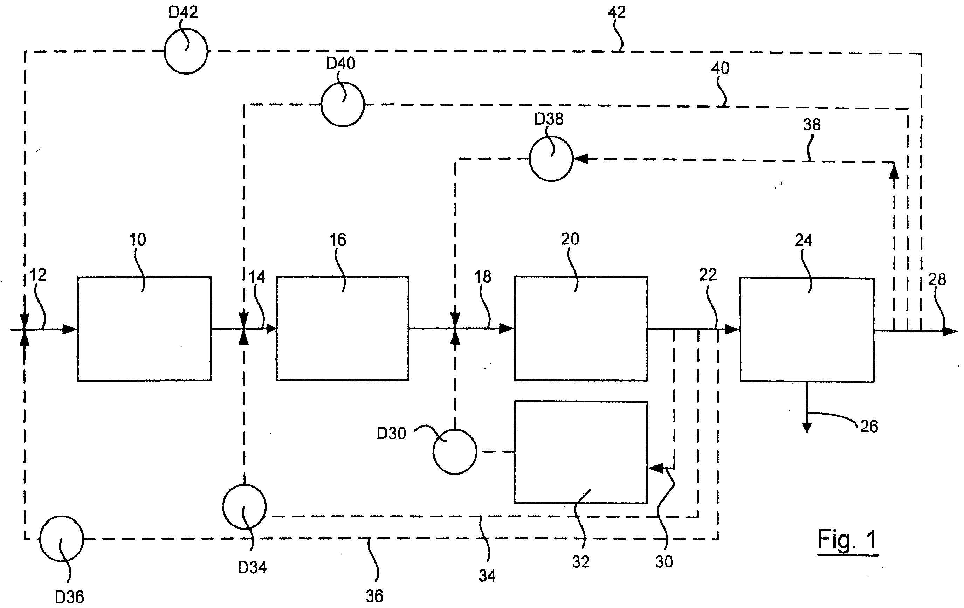

Einer Zerkleinerungsstation 10 wird gemäß Figur 1 ein Strom 12 aufzuschließenden trockenen Pflanzenmateriales zugeführt. Es kann sich hierbei um getrocknete Lindenblüten, getrocknete Pfefferminze oder andere getrocknete Blüten oder Blätter von Heilpflanzen handeln. Es kann sich hierbei aber auch um andere getrocknete Pflanzenteile handeln, z.B. getrocknete Wurzeln von Pflanzen (z.B. Hauhechelwurzeln). Das pflanzliche Material kann auch ein solches sein, welches von Hause aus nur sehr wenig Flüssigkeit enthält, z.B. Rinden, Holz, Fruchtkerne, Samenkörner oder dgl..

Die Zerkleinerungsstation 10 kann Mittel zum Schneiden, Schnitzeln, Mahlen, Quetschen oder Pressen des pflanzlichen Materiales umfassen. Durch einen Pfeil 14 ist ein Strom zerkleinerten pflanzlichen Materiales angedeutet. Dieser wird einem Vorbehandlungsreaktor 16 zugeführt, der eine schwach leitfähige Flüssigkeit, insbesondere Wasser enthält.

Das gemahlene pflanzliche Material bleibt im Kontakt mit dem Wasser ausreichend lange im Vorbehandlungsreaktor 16; so lange bis Wasser in das Innere der Zellen eingedrungen ist. Dieses Eindringen von Wasser kann man dadurch unterstützen, daß man den Inhalt des Vorbehandlungsbehälters 16 auf eine Temperatur einstellt, die bei druckfreiem Vorbehandlungsbehälter und Verwendung von Wasser als Penetrationsflüssigkeit zwischen 5°C und 95° C liegen kann.

Alternativ kann man das Einbringen von Wasser in die Zellen des pflanzlichen Materiales mit überhitztem Wasserdampf vornehmen, wobei eine Temperatur von bis etwa 150° C aus apparativen Gründen und Gründen des Schutzes des biologischen Materiales bevorzugt werden.

Nochmals im Hinblick auf die Schonung des pflanzlichen Materiales und insbesondere von in diesem enthaltenden Eiweißen wird eine Temperatur des Wasserdampfes von etwa 50°C bis etwa 80°C bevorzugt, wobei man vorteilhaft auch den Druck des Wasserdampfes so erniedrigt, daß das Wasser bei der genannten Temperatur siedet. Dies bedeutet bei einer Temperatur von 80°C einen Druck von etwa 0,47 bar, bei 70°C einen Druck von 0,32 bar und bei 50°C einen Druck von 0,12 bar.

Es sei darauf hingewiesen, daß die Arbeitstemperatur und Behandlungsdauer im Vorbehandlungsreaktor 16 im Hinblick darauf gewählt wird, das Eindringen von Wasser ins Innere der Zellen zu begünstigen, nicht aber im Hinblick darauf, aus den Zellen Wirkstoffe herauszuwaschen.

Nach ausreichender Verweilzeit im Vorbehandlungsreaktor 16 wird ein mit 18 symbolisch dargestellter Maischestrom aus zerkleinertem wieder mit Wasser angereichertem biologischen Material und Wasser in einen Elektroporationsreaktor 20 überführt. Dort wird die Maische einer Aufeinanderfolge sehr kurzer hoher Feldimpulse ausgesetzt, deren Größe so bestimmt ist, daß man über eine Zelle einen Spannungsabfall von etwa 0,5 bis 2 V erhält.

Bei Zellabmessungen im Bereich von einigen *µ*m bis einigen 100 *µ*m sowie bei gängigen Leitfähigkeiten von pflanzlichen Maischen von einigen zig-*µ*S/cm bis einigen zig-mS/cm bedeutet dies, das Erzeugen elektrischer Feldstärken im Inneren des Elektroporationsreaktors im Bereich zwischen 1 kV/cm und 100 kV/cm.

Im einzelnen wird man die Feldstärke je nach dem aufzuschließenden Material wählen. Dabei kann man in kleinerem Ausmaße auch durch Vergrößerung der Länge der einzelnen Feldimpulse einen fehlenden Amplitudenteil der Feldimpulse ausgleichen, was die Verwendung eines einfacheren und preisgünstigeren Impulsgenerators ermöglicht.

Am Ausgang des Elektroporationsreaktors 20 erhält man somit einen Materialstrom 22, der nun als Trägermedium dienendes übriges Penetrationswasser, Zellwandhüllen und Zellinhalte umfasst.

Der aufgeschlossene Materialstrom 22 kommt ggf. nach Abtrennung des Trägerwassers in eine Extraktionsstation / Nachbehandlungsstation 24, in welcher unter Verwendung eines geeigneten Lösungsmittels und/oder physikalisch (mechanisch und/oder unter Druckeinwirkung und/oder Erwärmung) der gewünschte Wirkstoff aus dem Zellinhalt und/oder den Zellwänden herausgelöst wird (durch die Zerstörung der Zellwände werden auch an und/oder in diesen gelagerte Stoffe für eine Extraktion besser zugänglich) .

Ein Extraktstrom 26 kann von der Extraktionsstation 24 abgezogen werden, während ein Material-Reststrom 28 entsorgt wird, der das Trägerwasser und in diesem verbliebene nicht extrahierte Anteile des Materialstromes 22 umfasst.

Das oben beschriebene Verfahren kann chargenweise oder kontinuierlich durchgeführt werden, wobei die verschiedenen Reaktoren mit kontinuierlich arbeitenden Drehschleusen oder entsprechenden intermittierend betätigten Ventilen versehen werden, was hier nicht im einzelnen erläutert zu werden braucht.

Auch tierisches Material, aus dem Wirkstoffe gewonnen werden sollen, wird oft zunächst getrocknet, um es vor der Verarbeitung länger lagern oder über größere Strecken preisgünstig transportieren zu können. Es kann dann genau so verarbeitet werden, wie oben unter Bezugnahme auf pflanzliche Materialien beschrieben.

Bei den oben beschriebenen Verfahrensbeispielen handelte es sich um solche, bei denen organische Wirkstoffe aus den Ausgangsmaterialien gewonnen werden sollten. Es versteht sich, daß man dieses Verfahren auch gleichermaßen dazu verwenden kann, anorganische Stoffe aus biologischem Material zu gewinnen ("leaching").

Bei den oben beschriebenen Verfahren lag auch das Interesse an den aus dem Ausgangsmaterial gewonnenem Wirkstoffen. Man kann das erfindungsgemäße Verfahren aber auch als Reinigungsverfahren verwenden, bei welchem aus einem Ausgangsmaterial ein unerwünschter löslicher Schadstoff entfernt wird. In diesem Falle wird dann der Extraktstrom 26 verworfen, während der Materialreststrom 28 einer weiteren Verwertung zugeführt wird.

Abwandlungen des oben beschriebenen Verfahrens sind in Figur 1 durch gestrichelte Linien dargestellt.

Erste Variationen des Verfahrens beziehen sich darauf, daß zumindest ein Teil des Materialstromes 22 wieder einer vorhergehenden Verfahrensstufe zugeführt wird.

Für manche Materialien kann es vorteilhaft sein, einen vollständigen Aufschluß des Materiales nicht in einem einzigen Durchlauf durch den Elektroporationsreaktor 20 zu bewerkstelligen, da dieser dann zu viel Zeit (und Energie) benötigen würde. Der Materialstrom wird daher gemäß einer Variante auf einem Weg 30 über eine Zwischenbehandlungsstation 32 zum Eingang des Elektroporationsreaktors 20 zurückgeführt.

Die Zwischenbehandlung 32 kann einfach eine Lagerung des Elektroporations-behandelten Materialstromes in einem Behälter für eine vorgegebene Zeitspanne beinhalten, bei der durch weiteres Quellen ausgehend von schon erzeugten Perforationen neue Angriffspunkte für einen weiteren Elektroporationsschritt geschaffen werden.

Die Zwischenbehandlung kann aber auch in einer chemischen Behandlung bestehen, welche ausgehend von dem im ersten Elektroporationsschritt erzeugten Fehlern in den Zellwänden neue Angriffspunkte für eine folgende weitere Elektroporation bilden.

Auch kann man dadurch, daß man ins Innere der Zellen über bei der ersten Elektroporation erzeugte Zellwandfehler eine stärker elektrisch leitende Flüssigkeit eindiffundieren läßt, bei einer weiteren Elektroporation, bei welcher weiterhin schlecht leitende Trägerflüssigkeit verwendet wird, den Strom durch den Reaktor stärker durchs Innere der einzelnen Zellen richten und so deren Zerstörung verbessern.

Bei einer weiteren Variante wird ein Teil des Materialstromes 22 über einen Weg 34 zum Einlaß des Vorbehandlungsreaktors 16 zurückgeführt. Man erhält so ein vollständigeres Wiederfüllen der einzelnen Zellen mit Flüssigkeit, da dies durch bei der ersten Elektroporation in der Wand erzeugte Fehler unterstützt wird.

Schließlich kann man einen Teil des Materialstromes 22 über einen Weg 36 auch zum Einlaß der Zerkleinerungsstation 10 zurückführen und so beim mechanischen Zerkleinern davon Gebrauch machen, daß das Material bei der ersten Elektroporation schon mechanisch geschwächt wurde. Man kann so das Material unter geringem Energieeinsatz auch sehr fein zerkleinern, was beim trockenen Material mit intakten Zellwänden schlechter möglich war.

Weitere Varianten des in Figur 1 gezeigten Verfahrens bestehen darin, daß man Teile des Materialreststromes 28 zu vorhergehenden Stufen des Verfahrens zurückführt. Diese Rückführungen sind durch Wege 38, 40, 42 angedeutet, die zum Elektroporationsreaktor 20, zum Anreicherungsreaktor 16 bzw. zur Zerkleinerungsstation 10 zurückführen. Die hiermit erhaltenen Vorteile liegen generell in einem intensiveren Aufschluß, ähnlich wie für die Wege 30, 34, 36 beschrieben.

Zur Steuerung der Durchsätze in den Wegen 30, 34, 36, 38, 40, 42 sind in diesen entsprechende Durchsatzregler D30, D34, D36, D38, D40, D42 vorgesehen. Diese können im konkreten Falle durch ein Proportionalventil gebildet sein, welches durch einen Servomotor bewegt wird, der seinerseits durch eine Steuerung aktiviert wird.

In weiterer Abwandlung des in Figur 1 gezeigten Verfahrens können auch in den Wegen 34 bis 42 Zwischenbehandlungen vorgesehen sein, um die Zellwände weiter zu schwächen und so die Trennung zwischen gewünschten und unerwünschten Bestandteilen des biologischen Materiales zu verbessern.

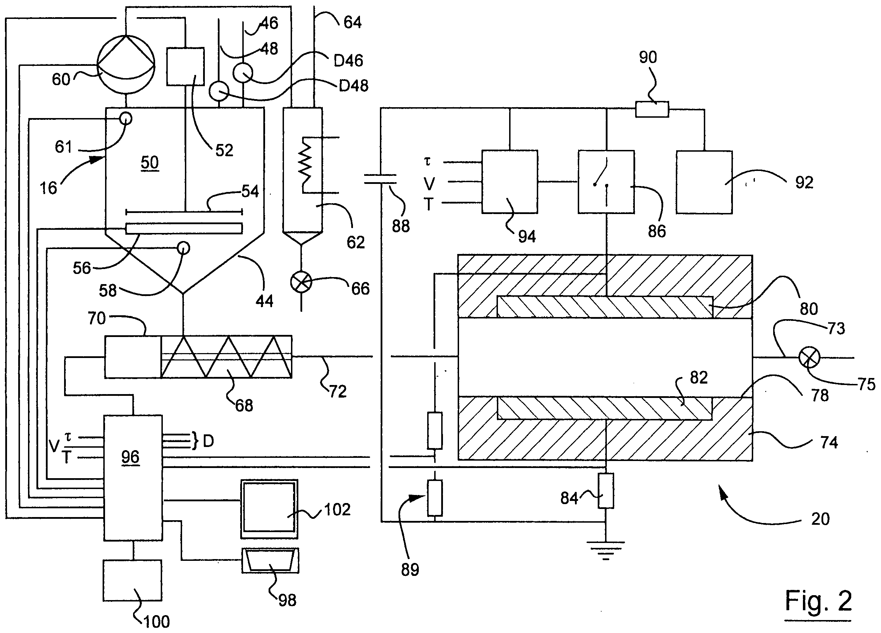

Figur 2 zeigt schematisch Einzelheiten des Vorbehandlungsreaktors 16 und des Elektroporationsreaktors 20 sowie einer zugehörigen Steuerung.

Der Vorbehandlungsreaktor 16 umfasst einen Behälter 44 mit kegelförmigem Auslaßabschnitt, der druckdicht abgeschlossen ist und dem über eine erste Leitung 46 Wasser und über eine zweite Leitung 48 das zerkleinerte biologische Material zugeführt wird, welchen die Zerkleinerungsstation 10 bereitstellt.

Steuerbare Durchsatzregler D46 und D48 dienen wieder zum Einstellen der Materialströme in den Leitung 46 und 48.

Im Inneren der Behälters 44 ist ein Volumen 50 aus Wasser und zerkleinertem biologischen Material schematisch dargestellt.

Ein Elektromotor 52 ist vorgesehen, um einen Rührer 54 zu drehen.

Im Behälter 44 ist ein Temperierstab 56 vorgesehen, um den Behälterinhalt auf eine von der Umgebungstemperatur abweichende Temperatur zu bringen, die typischerweise zwischen etwa 5°C und etwa 95°C, vorzugsweise zwischen etwa 15°C und etwa 50°C liegen kann.

Die im Behälter 44 herrschende Temperatur wird durch einen Temperaturfühler 58 gemessen.

Das Innere des Behälters 44 wird unter Verwendung einer Vakuum-Pumpe 60 evakuiert, um aus der Mischung aus Wasser und gemahlenem biologischen Material Restluftanteile abzusaugen. Ein Drucksensor 61 überwacht das Vakuum im Behälter 44.

Der Auslaß der Vakuum-Pumpe 60 ist über eine Kältefalle 62 mit einer Abluftleitung 64 verbunden. Das sich in der Kältefalle 62 ansammelnde Kondensat kann über ein Ventil 66 abgezogen werden.

Bei dem Kondensat kann es sich je nach verwendetem biologischem Material z.B. um leicht flüchtige ätherische Öle handeln, die einer Weiterverwendung zugeführt werden können.

Der Auslaß des Behälters 44 ist mit einer Schneckenpumpe 68 verbunden, die durch einen Elektromotor 70 angetrieben wird, der drehzahlsteuerbar ist.

Der Auslaß der Schneckenpumpe 68 ist mit dem Einlaß des Elektroporationsreaktors 20 durch eine Leitung 72 verbunden. Dort hält die Schneckenpumpe 68 einen atmosphärischen Druck oder einen Überdruck aufrecht, wodurch gewährleistet ist, daß im Elektroporationsreaktor 20 unter den dort herrschenden elektrischen Feldern die Zahl durch Gaseinschlüsse bedingter Spannungsüberschlägen klein gehalten wird.

Um den im Inneren des Elektroporationsreaktors 20 durch die Schneckenpumpe 68 eingestellten Druck an Atmosphärendruck (oder sonstigen Folgedruck) heranzuführen, ist in einer das elektroporierte Material abführenden Auslaßleitung 73 eine Zellenradschleuse 75 vorgesehen. Alternativ ist zur Druckerniedrigung eine Drossel, ein Durchsatzregler oder ein Ventil verwendbar, zu einer weiteren Druckerhöhung eine weitere Pumpe.

Der Elektroporationsreaktor 20 hat ein aus durchschlagfestem isolierendem und schlagzähem Material gefertigtes Gehäuse 74, welches einen flachen Reaktionskanal 78 mit rechteckigem Querschnitt aufweist. In der Praxis kann die Höhe des Reaktionskanales 78 etwa 5 mm bis 200 mm, vorzugsweise etwa 40 mm betragen.

Als Material für das Gehäuse 74 kommen insbesondere in Frage: Kunststoffe und faserverstärkte Kunststoffe, insbesondere transparente oder transluzente Kunststoffe wie Polycarbonat; Glas, insbesondere Verbundglas; hochfeste Industriekeramik, ggf. auch faserverstärkt.

In die in der Zeichnung obere und untere Begrenzungsfläche des Reaktionskanales 78 sind Elektroden 80, 82 bündig eingesetzt, die als Metallplatten ausgebildet sind. Vorzugsweise umfaßt das Plattenmaterial (ggf. als Beschichtung) Edelstahl, Titan oder Titan-Iridium, Edelmetall, insbesondere Gold, Silber Platin.

Die Elektroden 80, 82 sind an den Kanten großzügig abgerundet, um dort Feldspitzen zu vermeiden.

Von diesen ist die untere Elektrode 82 über einen Meßwiderstand 84 mit Erde verbunden, während die obere Elektrode 80 über einen steuerbaren Schalter 86 mit einer Kondensatorbatterie verbunden ist, die als einziger Kondensator 88 dargestellt ist.

Ein Spannungsteiler 89 gestattet die Messung der an den Elektroden 80, 82 liegenden Spannung und in Verbindung mit dem durch den Meßwiderstand 84 gebildeten Stromfühler die Messung der elektrischen Leistung.

Das Aufladen des Kondensators 88 erfolgt über einen Ladewiderstand 90 durch eine Hochspannungs-Ladeeinheit 92. Diese braucht für kleinere Elektroporationsreaktoren, wie sie in Verbindung mit dem Aufschließen von Heilpflanzen Verwendung finden können, nur Ströme von einigen mA bis zu einigen A bereitzustellen.

Zur Steuerung des Schalters 86 ist ein Steuerkreis 94 vorgesehen, der an zwei Eingangsklemmen ein Signal *τ*, welches die Länge eines Schaltimpulses vorgibt, sowie ein Signal T erhält, welches den zeitlichen Abstand von Schaltimpulsen vorgibt.

Zusätzlich kann der Steuerkreis 94 eine Steuerklemme V aufweisen, an welcher vorgebbar ist, bei welcher Spannung der Schalter 86 betätigt werden soll.

Die Steuersignale *τ*, T und V werden von einem Prozeßrechner 96 bereitgestellt. Wie durch ein Leiterbündel D angedeutet, besorgt der Prozessrechner 96 auch die Steuerung der verschiedenen Durchsatzregler D, die in Leitungen der Anlage vorgesehen sind, wie oben beschrieben.

Dem Prozessrechner 96 können über ein Tastenfeld 98 Kennwerte für das jeweils aufzuschließende biologische Material eingegeben werden. Unter Verwendung dieser Kennwerte und diesen zugeordneter Sätze von Arbeitsparametern, die in einem Speicher 100 des Prozeßrechners 96 abgelegt sind, berechnet der Prozeßrechner 96 die jeweils für das betrachtete biologische Material günstigsten Werte für die Spannung, auf welche der Kondensator 88 aufgeladen werden soll, für die Breite *τ* der Schaltimpulse und für den Abstand T der Schaltimpulse sowie für weitere Arbeitsparameter der Elektropoarationsanlage inkl. des Vorbehandlungsreaktors 16 und anderer in Figur gezeigter Anlagenteile.

Ein Monitor 102 dient der Anzeige von Betriebsparametern der Anlage und der Kontrolle von Eingaben.

Typische Werte für *τ* sind etwa 10 ns bis etwa 100 *µ*s, vorzugsweise etwa 100 ns bis etwa 50 *µ*s.

Typische Werte für 1/T sind etwa 0,1 Hz bis etwa 500 Hz, vorzugsweise etwa 0,2 Hz bis etwa 50 Hz, nochmals bevorzugt etwa 0,5 Hz bis etwa 40 Hz.

Jedes Mal, wenn ein durch einen Schaltimpuls der Schalter 86 geschlossen wird, entlädt sich der Kondensator 88 über das Maischevolumen, welches sich gerade zwischen den beiden Elektroden 80 und 82 befindet. Dabei treten kurzfristig Ströme im Bereich von einigen Tausend A auf.

Durch diese Ströme wird unter Berücksichtigung der elektrischen Leitfähigkeit der Maische zwischen den oberen und unteren Abschnitten der Zellwand einer Zelle Spannungsunterschiede im Bereich von etwa 2 V erzeugt. Diese Unterschiede sind so groß, daß in den Zellwände schon bestehende kleine Poren und Ionenkanäle irreversibel aufgeweitet werden und die Zellwand so zerstört wird.

Wenn die Leitfähigkeit der Maische zu klein ist, ist der Strom, der beim Schließen des Schalters 86 durch die Maische fließt, zu klein. Ist umgekehrt, die elektrische Leitfähigkeit der Maische zu groß, so wird wegen der guten Leitfähigkeit der Maische an den einzelnen Zellen kein so großer Spannungsabfall aufgebaut, daß eine Zerstörung der Zellwände stattfindet.

Aus diesem Grunde muß die Leitfähigkeit der Maische in einem bestimmten Fenster liegen, dessen untere Grenze bei etwa 0,1 mS/cm und dessen obere Grenze bei etwa 100 mS/cm liegt.

Um die Leitfähigkeit der Maische laufend zu überwachen, ist der Meßwiderstand 84 vorgesehen, der mit einem Eingang des Prozeßrechners 96 verbunden ist. Alternativ oder zusätzlich kann auch der Spannungsteiler 89 verwendet werden.

Der Prozeßrechner 96 ändert bei Feststellung einer Leitfähigkeitsänderung sofort die Spannung, auf die der Kondensator 88 aufgeladen wird derart, daß man wieder den gleichen Spannungsabfall an einer Zelle erhält, so daß das Aufbrechen der Zellwände weiterhin sicher erfolgt. Diese Maßnahme führt aber zu einer stärkeren Belastung der Hochspannungs-Ladeeinheit 92.

Gleichzeitig wird der Prozeßrechner 96 daher die Herstellung der Maische so beeinflussen (über die Steuerung der Temperatur, des Rührens und die Mengen zugeführten biologischen Materiales und Wassers), daß die elektrische Leitfähigkeit der Maische wieder in den normalen Bereich zurückkehrt. In dem Maße, wie dies erreicht wird, kann dann die Aufladespannung des Kondensators 88 wieder auf den Ausgangwert hin zurückgefahren werden.

Die der Fließgeschwindigkeit des Trägerwassers entsprechende Fördergeschwindigkeit der Zellen durch den Elektroporationsreaktor ist so bemessen, daß die Zellen einer ausreichenden Anzahl von Feldimpulsen ausgesetzt sind, um die Zellwände so stark zu zerstören, daß aus dem Inhalt der Zelle bzw. der Zellwände in einem nachfolgenden Behandlungsschritt mit einer gewünschten Ausbeute und einer gewünschten Extraktionszeit Wirkstoffe entnommen werden können.

Die Gesamtzahl der Feldimpulse, denen das Zellmaterial im Elektroporationsreaktor 20 ausgesetzt ist, die Breite und Frequenz der Feldimpulse und deren Höhe, die Temperatur beim Elektroporieren sowie die Arbeitsparameter beim Vorbereiten der Maische (Zerkleinerungsgrad des trockenen biologischen Materiales, Mischungsverhältnis mit Wasser, etwa verwendete Vorbehandlungsadditive, Temperatur bei der Vorbehandlung, Dauer der Vorbehandlung, Zwischenlagerzeiten) werden jeweils für eine bestimmtes für einen bestimmten Zweck aufzuschließenden Material experimentell bestimmt und im Speicher 100 zum Abruf über das Tastenfeld 98 bereitgehalten. Dabei kann man von Parametern ausgehen, die für von der Zellgröße und von der Zusammensetzung her ähnlichen Zellen früher bestimmt wurden.

In Abwandlung des oben beschriebenen Ausführungsbeispieles kann man den Schalter 86 durch eine Gasentladungsstrecke ersetzen. Dabei macht man von der Eigenschaft einer Gasentladungsstrecke Gebrauch, daß diese bei Anlegen einer vorgegebenen Spannung durchbricht. Diese Durchbruchspannung kann man über den Druck des in der Gasentladungsstrecke eingeschlossenen Gases steuern. Eine Drucksteuerung für die Gasentladungsstrecke ersetzt somit im Effekt den Steuerkreis 94.

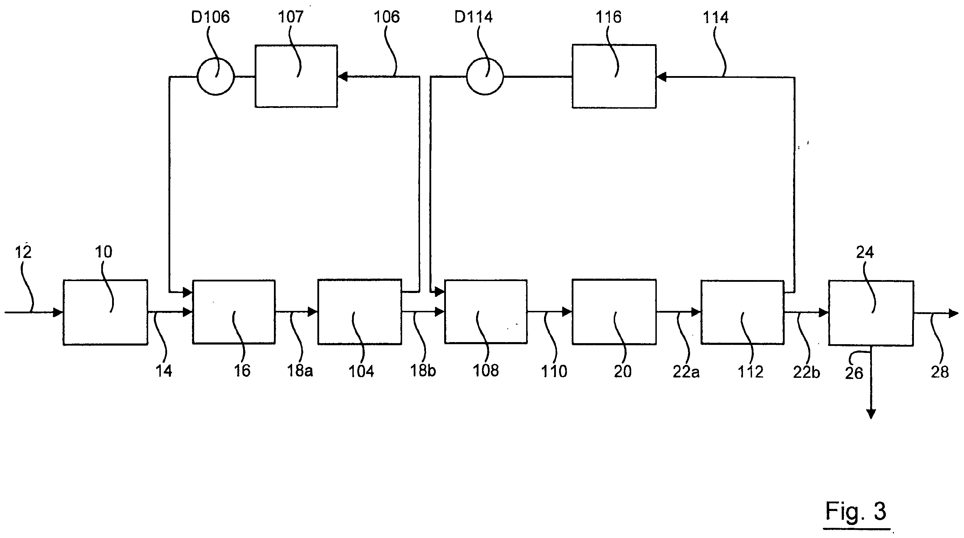

Das in Figur 3 veranschaulichte Verfahren unterscheidet sich von demjenigen in Figur 1 dadurch, daß zum Wiederherstellen einer Leitfähigkeit im Inneren der Zellen eine Penetrationsflüssigkeit verwendet wird, die sich von der Trägerflüssigkeit unterscheidet, die dazu dient, die Zellen durch den Elektroporationsreaktor zu tragen.

Komponenten der in Figur 3 gezeigten Anlage, die schon unter Bezugnahme auf Figur 1 besprochen wurden, sind wieder mit denselben Bezugszeichen versehen und brauchen nicht nochmals detailliert beschrieben zu werden. Auch sind in Figur 3 die verschiedenen Varianten einer Produktrückführung im Prozeß der Übersichtlichkeit halber weggelassen. Es versteht sich, daß Rückführwege, welche den Wegen 30, 34, 36, 38, 40 und 42 von Figur 1 entsprechen, auch bei der Anlage nach Figur 3 vorgesehen werden können.

Die Anlage nach Figur 3 unterscheidet sich von derjenigen nach Figur 1 dadurch, daß ein gesonderter Trägerflüssigkeitsstrom verwendet wird, um die Partikel des aufzuschließenden Biomateriales durch den Elektroporationsreaktor 20 hindurchzutragen.

Insbesondere kann als Penetrationsflüssigkeit Wasser verwendet werden, dem die Leitfähigkeit erhöhende Substanzen, insbesondere Salze zugefügt sind, während als Trägerflüssigkeit normales Leitungswasser mit geringerer Leitfähigkeit oder deionisiertes oder destilliertes Wasser verwendet wird.

Die Trägerflüssigkeit kann auch eine solche sein, die sich mit der im Vorbehandlungsreaktor 16 verwendeten Penetrationsflüssigkeit nicht mischt. Vorzugsweise wird in dem Vorbehandlungsreaktor 16 Wasser oder eine wässrige Lösung verwendet. Eine solche Penetrationsflüssigkeit kann in der Regel die Zellwände gut durchdringen, wobei ggf. eine oberflächenaktive Substanz zugemischt werden kann, um von den Zelloberflächen getragene Wachsschichten oder Fettschichten anzugreifen. Als mit Wasser nicht mischbare Trägerflüssigkeit wird dann ein im angegebenen Leitfähigkeitsbereich von 0,1 mS/cm bis 10 mS/cm leitendes Öl verwendet.

Auch Gase kommen als Trägermedium in Frage, sofern sie schwach leitend und durchschlagfest sind. Um eine Schwerkraftentmischung von Trägermedium und biologischem Material zu vermeiden, wird dabei der Elektroporationsreaktor 20 vertikal durchströmt. Gleiches gilt für vorgeschaltete und nachfolgende Anlagenteile.

An den Ausgang des Vorbehandlungsreaktors 16 ist eine Trennstufe 104 angeschlossen, welche die nicht vom biologischen Material aufgenommene Penetrationsflüssigkeit abtrennt. Die Trennstufe 104 kann z.B. ein Filter oder eine Zentrifuge sein. Die dort abgetrennte Penetrationsflüssigkeit kann über eine Leitung 106, die eine Regenerationseinheit 107 enthalten kann, in den Vorbehandlungsreaktor 16 zurückgeführt werden.

Ein steuerbarer Durchsatzregler D106, der ähnlichen Aufbau aufweisen kann, wie die unter Bezugnahme auf die vorstehenden Figuren beschriebenen Durchsatzregler, gibt die Größe des Materialstromes in der Leitung 106 vor.

Ein dem Maischestrom 18a entsprechender, jedoch nur noch wenig Penetrationsflüssigkeit enthaltender Strom 18b aus wieder mit Flüssigkeit angereichertem biologischen Material wird dem einen Eingang einer Mischstufe 108 zugeführt, deren anderer Eingang das Trägermedium erhält. Am Ausgang der Mischstufe 108 erhält man einen Materialstrom 110 aus Trägermedium und Biomaterial, dessen Zellen wieder mit Wasser gefüllt sind.

Der Materialstrom 110 durchläuft dann den Elektroporationsreaktor 20, wobei die Zellwände des Materiales wieder durch die scharfen und hohen Feldimpulse zerstört werden.

Der am Ausgang des Elektroporationsreaktors 20 erhaltene Materialstrom 22 wird dann in eine weitere Trennstufe 112 geführt, in welcher das Trägermedium einerseits und die zerstörten Zellen des biologischen Materiales andererseits voneinander getrennt werden. Hierzu können wieder bekannte physikalische Verfahren verwendet werden, z.B. Filtration oder Zentrifugieren.

Das Trägermedium wird über eine Leitung 114 und eine Regenerationsstufe 116 wieder zur Mischstufe 108 zurückgeführt, während der Strom von Zellwänden und Zellinhalten der Extraktionsstation 24 zugeführt werden.

Ein steuerbarer Durchsatzregler D114 gibt vor, wie viel Mischung über die Leitung 114 zurückgeführt wird.

Auf diese Weise ist es möglich, unterschiedliche Leitfähigkeiten im Trägermedium und im Inneren der zu zerstörenden Zellen einzustellen, derart, daß der zwischen den Elektroden der Elektroporationsstation fließende Strom bevorzugt durch die Zellen fließt, wo solche zur Verfügung stehen.

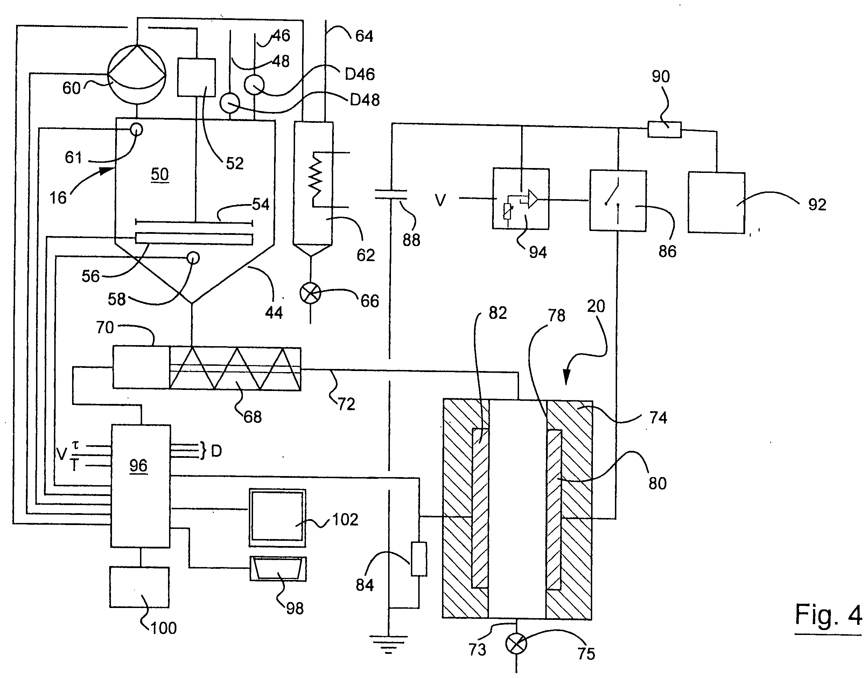

Beim Ausführungsbeispiel nach Figur 4, welches demjenigen nach Figur 2 sehr ähnlich ist, ist der Elektroporationsreaktor 20 mit vertikaler Ausrichtung des Behandlungskanales 78 aufgestellt.

Beim Ausführungsbeispiel nach Figur 4 wird der Schalter 86 durch einen Steuerkreis 94 geschaltet, der in Abhängigkeit von der Spannung am Kondensator 88 arbeitet. Der Steuerkreis 94 ist schematisch als Komparator mit einstellbarer Vergleichsspannung V wiedergegeben.

Erreicht beim Ausführungsbeispiel nach Figur 4 die Spannung am Kondensator 88 einen vorgegebenen Wert, so schließt der Steuerkreis 94 den Schalter 86, und die auf dem Kondensator gespeicherte Ladung fließt impulsförmig über die Elektroden 80, 82 und die zwischen diesen befindliche Mischung sowie den Messwiderstand 84 gegen Erde ab. Der Schalter 86 wird dann wieder geöffnet und der Kondensator 88 wird von der Ladeeinheit 92 über den Ladewiderstand 90 wieder aufgeladen. Die gesamte Anordnung arbeitet somit als Hochspannungs-Impulsgenerator.

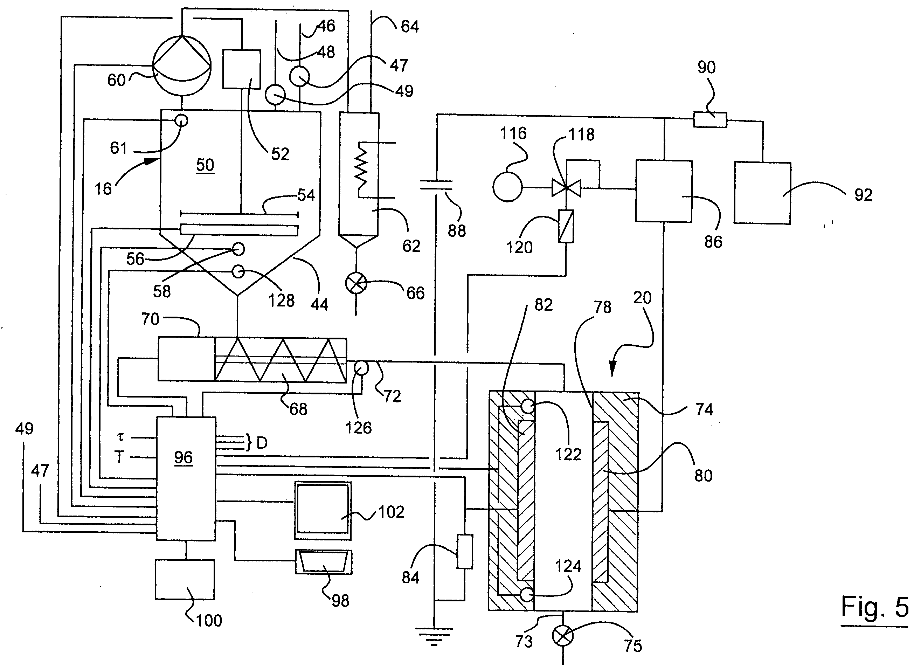

Beim Ausführungsbeispiel nach Figur 5 ist der Schalter 86 durch eine Entladungsstrecke 86' ersetzt. Diese schlägt jeweils durch, wenn die Spannung an ihren Elektroden einen vorgegebenen Wert überschreitet. Die Durchschlagsspannung hängt von der Art des Gases, welches sich in der Durchschlagsstrecke 86' befindet und von dessen Druck ab.

Hierzu ist ein Vorratsbehälter 116 vorgesehen, welcher einen Vorrat des in der Durchschlagstrecke 86' verwendeten Arbeitsgases enthält. Der Auslass des Vorratsbehälters 116 ist über einen steuerbaren Druckregler 118 mit der Durchschlagstrecke 86' verbunden, wobei die einstellbare Schließkraft durch einen Elektromagneten 120 veranschaulicht ist, der von dem Prozessrechner 96 her bestromt wird.

In weiterer Abwandlung sind bei der Anlage nach Figur 5 im Gehäuse 74 ein Lichtdetektor 122, und ein Drucksensor (oder Mikrofon) 124 vorgesehen, deren Ausgangssignale ODER-mäßig zusammengeschaltet sind und auf einen Eingang des Prozessrechners 96 gegeben werden.

Erfolgt in dem Reaktionskanal 78 ein Durchschlag, so wird der entsprechende Lichtblitz bzw. die entsprechende Druck- oder Schallwelle von den Detektoren 122, 124 registriert, und der Prozessrechner 96 kann dann Abhilfe schaffen, wie später noch genauer beschrieben wird.

Schließlich ist bei der Anlage gemäß Figur 5 am Auslass der Schneckenpumpe 68 ein Leitfähigkeitssensor 126 vorgesehen, der mit dem Prozessrechner 96 verbunden ist.

Einen weiteren Leitfähigkeitssensor 128 kann man im Inneren des Behälters 44 vorsehen.

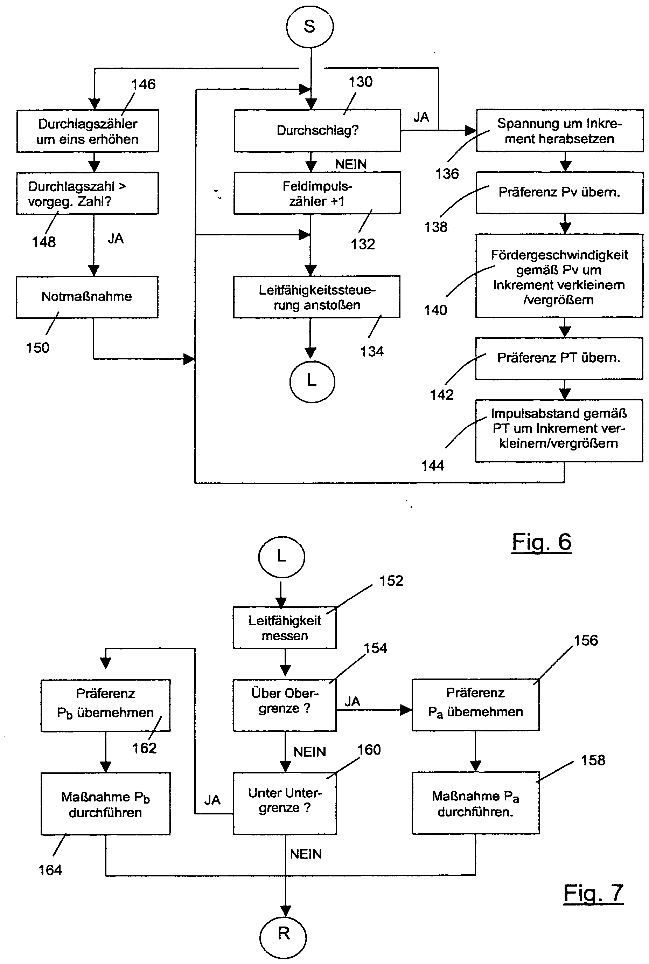

Unter Bezugnahme auf die Figuren 6 und 7 soll nun das Arbeiten des Prozessrechners 96 skizziert werden, soweit es dazu dient, im Elektroporationsreaktor 20 gleichbleibend gute Arbeitsverhältnisse aufrecht zu erhalten.

Hierzu überwacht der Prozessrechner 96 zum einen die Leitfähigkeit der Mischung aus Trägermedium und biologischem Material vom Behälter 44 bis ins Innere des Reaktionskanales 78 (dort über die Messung der Stromimpulse, die über den Messwiderstand 84 laufen).

Außerdem überwacht der Prozessrechner 96, ob im Inneren des Reaktionskanales 78 Spannungsüberschläge vorkommen.
Er kann dies anhand der Lichtimpulse tun, die auf den Lichtsensor 122 fallen, anhand von Druckimpulsen oder Schallimpulsen, welche den Drucksensor bzw. das Mikrofon 124 erreichen, und auch anhand der Breite der Stromimpulse, welche durch den Messwiderstand 84 fließen, und/oder der Spannungsimpulse, welche der Spannungsteiler 89 bereitstellt.

Grundsätzlich arbeitet eine Hauptroutine des Prozessrechners 96 in zwei Stufen unterschiedlicher Priorität: Durchschlagsüberwachung (übergeordnet) und Leitfähigkeitsregelung (nachgeordnet).

Der Prozessrechner 96 arbeitet dabei getaktet durch Impulse, die auch zur Ansteuerung des Steuerkreises 94 dienen bzw. von der am Messwiderstand 84 abfallenden Spannung und/oder der Ausgangsspannung des Spannungsteilers 89 abgeleitet sind.

Zunächst wird in einem Block 130 geprüft, ob die jeweils angelegte Spannung zu einem Durchschlag geführt hat.

Ist dies nicht der Fall, wird ein Feldimpulszähler (Block 132) um eins hochgesetzt. Anschließend wird die Leitfähigkeitssteuerung angestoßen (Block 134), die in Figur 7 näher dargestellt ist.

Wird ein Durchschlag festgestellt, so wird zunächst in einem Block 136 die Spannung herabgesetzt auf welche der Kondensator 88 aufgeladen wird, bzw. bei welcher der Schalter 86 geschlossen wird. Dies kann durch Änderung des Referenzsignales für den Steuerkreis 94 bzw. der Bestromung des Elektromagneten 120 erfolgen. Die Spannung wird bei jedem auftretenden Durchschlag um ein vorgegebenes Inkrement herabgesetzt, welches erfahrungsgemäß dazu ausreicht, bei einem nächsten Feldimpuls das Auftreten eines Durchschlages zu vermindern.

In einem nächsten Schritt wird in einem Block 138 ein Präferenzwert für das weitere Vorgehen Pv von einem übergeordneten Programmteil übernommen. Dieser dient zum Aussuchen einer von einer Mehrzahl von Maßnahmen, die ebenfalls dazu dienen können, das Auftreten eines Durchschlages im Reaktorkanal 78 zu vermeiden.

Hierzu kann gehören, die Fördergeschwindigkeit v der Mischung kurzfristig zu erhöhen, um einen zu gut leitenden inhomogenen Bereich der Mischung aus dem Elektroporationsreaktor auszutragen, bevor der nächste Hochspannungsimpuls angelegt wird. Ist für das übergeordnete Programm erkennbar, dass es sich um einen voraussichtlich singulären Durchschlag handelte, so kann das übergeordnete Programm gerade entgegengesetzt die Verkleinerung der Fördergeschwindigkeit für sachdienlich halten, damit das im Reaktorkanal 78 befindliche Material einem zusätzlichen Hochspannungsimpuls ausgesetzt ist und somit beim Verlassen des Elektroporationsreaktors die Standard-Impulszahl erfahren hat.

Nach der im Block 140 erfolgten Änderung der Fördergeschwindigkeit (oder Beibehaltung der Fördergeschwindigkeit) wird in einem weiteren Block 142 von einem übergeordneten Programm ein weiterer Präferenzwert PT übernommen, der angibt, ob der Abstand T, in welchem der nächste Hochspannungsimpuls an den Durchflussreaktor 20 angelegt wird, schneller oder langsamer bereitgestellt wird. Diese Modifizierung des Impulsabstandes erfolgt im Block 144.

Dann erfolgt von dort ein Rücksprung zum Startpunkt S der Hauptroutine.

Wird ein Durchschlag festgestellt, so wird zugleich im Block 146 ein Durchschlagszähler um eins erhöht. Dieser wird in regelmäßigen Abständen durch einen anderen Programmteil zurückgesetzt, wie durch eine Rückstellklemme R angedeutet.

In einem Block 148 wird festgestellt, ob der Stand des Durchschlagszählers größer ist als eine vorgegebene Zahl (1, 2, 3, ...). Ist dies der Fall, wird in einem Block 150 eine Notfallmaßnahme getroffen.

Dies wird dann aus der nachstehenden Gruppe ausgewählt sein: Alarm und/oder Fehlerhalt der gesamten Anlage; Unterbinden weiterer Hochspannungsimpulse für eine vorgegebene Zeit; rasches Erniedrigen der Leitfähigkeit der im Reaktorkanal 78 befindlichen Mischung durch rasches Zuführen einer größeren Menge reinen Wassers; rasches Ausstoßen des Inhaltes des Reaktorkanales 78.

Figur 7 zeigt ein Unterprogramm, welches der Prozessrechner 96 dazu verwendet, die Leitfähigkeit der im Reaktorkanal 78 befindlichen Mischung innerhalb eines vorgegebenen Leitfähigkeitsbereiches zu halten, der durch eine Obergrenze und eine Untergrenze vorgegeben ist.

In einem Block 152 erfolgt die laufende Leitfähigkeitsmessung im Reaktorkanal 78.

In einem weiteren Block 154 wird geprüft, ob die aktuelle Leitfähigkeit zu groß ist. Ist dies der Fall, wird in einem Block 156 ein Präferenzwert Pₐ für eine durchzuführende Abhilfemaßnahme übernommen. Gemäß diesem Präferenzwert wird dann im Block 158 eine zugeordnete Maßnahme durchgeführt. Diese kann darin bestehen, den Anteil des biologischen Materiales in der Mischung herabzusetzen, die Intensität einer Vorbehandlung herabzusetzen, die Zugabe eines leitfähigen Additives herabzusetzen oder mehr Trägerwasser zuzusetzen.

Wird im Block 154 festgestellt, dass die Leitfähigkeit nicht zu groß ist, wird in einem Block 160 geprüft, ob die Leitfähigkeit zu klein ist.

Ist dies der Fall, so wird in einem Block 162 von einem übergeordneten Programmteil wieder ein Präferenzwert P_{b} übernommen, der angibt, welche einer Mehrzahl vorgesehener Abhilfemaßnahmen getroffen werden soll.

Diese Maßnahme kann aus nachstehender Gruppe ausgewählt sein: Erhöhen des Anteiles an biologischem Material in der Mischung, intensivere Vorbehandlung des biologischen Materiales, Zusatz eines leitfähigen Additives, Herabsetzen der Trägerwassermenge.

In einem Block 164 wird die entsprechende Maßnahme dann durchgeführt.

Ähnliche Regelschleifen können ständig ausgehend vom Ausgangswert des Leitfähigkeitsmessers 126 und des Leitfähigkeitsmessers 128 erfolgen.

Hat man eine Anlage mit Teilrückführung von Materialmengen, so kann die Einstellung der richtigen Leitfähigkeit in dem Reaktorkanal 78 auch dadurch erfolgen, dass man die Größe der rückgeführten Materialströme geeignet ändert.

Im Hinblick auf das Vermeiden von Durchschlägen im Elektroporationsreaktor ist es vorteilhaft, dort alle Arbeitsbedingungen zu meiden, die zu der Bildung von Gas- oder Dampfblasen führen könnten. Dies bedeutet auch, daß man im Elektroporationsreaktor einen höheren Druck einstellen wird als in vorgelagerten Behandlungsreaktoren, und daß man im Elektroporationsreaktor auch eine Temperatur einstellen wird, die höher ist als die in vorgelagerten Behandlungsreaktoren.

Ebenfalls im Hinblick auf das Vermeiden von Durchschlägen wird man bei einer Vorbehandlung des biologischen Materiales darauf achten, daß dieses keine Spitzen erhält bzw. vorhandene Spitzen abgerundet werden.

Das Extrahieren der Wirkstoffe oder Schadstoffe kann dann in der Extraktionsstufe 24 wieder auf die weiter oben geschilderte Weise erfolgen.

Die Extraktionsstufe ist nicht auf die Verwendung von extrahierenden Lösungsmittel beschränkt. Es können auch physikalische Extraktionsverfahren verwendet werden, die z.B. unter Druck- und/oder Temperatureinwirkung arbeiten.

Bei manchen Produkten kann es in weiterer Abwandlung der Erfindung auch von Interesse sein, ein von nicht erwünschten Stoffen befreites Produkt hinter der Extraktionsstufe 24 wieder zu trocknen. Als dann wieder flüssigkeitsarmes Produkt ist es auch zur Langzeitlagerung geeignet, ohne daß sich Mikroorganismen im Produkt vermehren. Das behandelte Produkt hat aber nach wie vor die Eigenschaft, daß es viele bei der Elektroporation entstandene Kanäle und freie Oberflächen aufweist, die zu einem späteren Zeitpunkt einen raschen Austausch von Wirkstoffen mit einer Flüssigkeit oder einem Gas ermöglichen.

So könnte man z.B. mit dem oben geschilderten Verfahren Kaffeebohnen behandeln, die sparsamer verwendet werden können, da eine intensivere Übertragung der Geschmacksstoffe und des Koffeins auf das Kaffeewasser möglich ist.

Zu typischen flüssigkeitsarmen biologischen Materialien, die mit dem oben beschriebenen Verfahren aufschließbar sind, gehören Vorprodukte für die Parfümindustrie (Rosenblätter, Lavendelblätter usw.), Vorprodukte für Drogerieartikel (Tannennadeln, Nadeln von Latschenkiefern usw.), Vorprodukte für die Pharmaindustrie (z.B. Blätter von Heilpflanzen (Eukalyptus, Pfefferminze, Melisse, Brennnessel, Lindenblüten), Wirkstoffe enthaltende Rinden usw. und auch Wurzeln, Blätter und Früchte von Arzneimittelpflanzen (Hauhechelwurzel, Tollkirsche, Fingerhut usw.) und Vorprodukte für die Lebensmittelindustrie (Gewürze, Trockenfrüchte, Linsen, Erbsen usw.)

Typische Beispiele für tierische biologische Materialien sind z.B. Fleisch (zur Gewinnung von Fleischextrakten) und Gewebe zur Hormongewinnung (Nebennierenrindenteile) usw..

Die nachstehenden Beispiele geben einen Überblick über zusammen mit bestimmten pflanzlichen Materialien geeignete Arbeitsparameter des Elektroporationsreaktors.

### Beispiel 1: Herstellung von Hauhechelwurzelsaft

Hauhechelwurzeln wurden mechanisch auf Stückchen von etwa 5 mm Durchmesser zerkleinert.

So erhaltene Wurzelschnitzel wurden im Verhältnis 1:3 mit Wasser zu einer Maischelösung zusammengegeben.

Die Maischelösung wurde über 10 Minuten bei 19 °C gerührt.

Man erhält so eine vorbehandelte Maischelösung mit einer elektrischen Leitfähigkeit von 5,9 mS/cm.

Diese Maischelösung wurde in einen Elektroporationsreaktor gegeben, dessen rechteckige einander gegenüberliegende Elektroden einen Abstand von 20 mm und Kantenlängen von 4 cm bzw. 13 cm aufwiesen.

Die Maischelösung wurde bei einer Feldstärke von 12,5 kV/cm mit 15 Feldimpulsen behandelt. Deren Länge betrug 2 *µ*s.

Unmittelbar nach der Elektroporation wurde eine Leitfähigkeit der Maische von 6,9 mS/cm gemessen.

Nach Ruhen der Maische nach der Elektroporation über 3 Stunden wurde das Wasser abgeschüttet und der Wurzelsaft abgepresst und gefiltert.

Dessen Leitfähigkeit betrug 10,45 mS/cm.

Der Rückstand nach dem Abpressen betrug 6,15%.

### Beispiel 2: Herstellung von Lindenblütensaft

Lindenblüten mit einem Durchmesser von 5 bis 10 mm wurden im Verhältnis 1:2 mit Wasser zu einer Maischelösung zusammengegeben.

Die Maischelösung wurde über 10 Minuten bei 19 °C gerührt.

Man erhält so eine vorbehandelte Maischelösung mit einer elektrischen Leitfähigkeit von 3,3 mS/cm.

Diese Maischelösung wurde in einen Elektroporationsreaktor gegeben, dessen rechteckige einander gegenüberliegende Elektroden einen Abstand von 20 mm und Kantenlängen von 4 cm bzw. 13 cm aufwiesen.

Die Maischelösung wurde bei einer Feldstärke von 11,5 kV/cm mit 15 Feldimpulsen behandelt. Die Länge der Feldimpulse betrug 2 *µ*s.

Unmittelbar nach der Elektroporation wurde eine Leitfähigkeit der Maische von 4,68 mS/cm gemessen.

Nach Ruhen der Maische nach der Elektroporation über 3 Stunden wurde das Wasser abgeschüttet und der Saft abgepresst.

Dessen Leitfähigkeit betrug 7,0 mS/cm.

Der Rückstand nach dem Abpressen des Saftes betrug 3,84%.

## Patentansprüche

1. Verfahren zum Aufschließen von biologischem Material, bei welchem
a) das biologische Material in einem Trägermedium verteilt wird und
b) die Mischung aus Trägermaterial und biologischem Material durch Elektroporation behandelt wird,
**dadurch gekennzeichnet, dass**
c) das Auftreten von elektrischen Durchschlägen bei der Elektroporationsbehandlung überwacht wird und
d) mindestens ein Parameter der Elektroporationsbehandlung in Abhängigkeit vom Ergebnis der Überwachung der gesteuert wird.

2. Verfahren nach Anspruch 1, **dadurch gekennzeichnet,**
**dass** man das Auftreten von Durchschlägen anhand von bei den Durchschlägen entstehendem Licht überwacht.

3. Verfahren nach Anspruch 1 oder 2, **dadurch gekennzeichnet, dass** man das Auftreten von Durchschlägen anhand von bei den Durchschlägen entstehenden Druckstößen und/oder von bei den Durchschlägen entstehendem Schall überwacht.

4. Verfahren nach einem der Ansprüche 1 bis 3, **dadurch gekennzeichnet, dass** man die Durchschläge anhand der bei der Elektroporation durch die Mischung fließenden Stromimpulse und/oder anhand der an der Mischung abfallenden Spannungsimpulse überwacht.

5. Verfahren nach Anspruch 4, **dadurch gekennzeichnet, dass** man die Amplitude und/oder die Länge der bei der Elektroporation fließenden Stromimpulse und/oder der an der Mischung abfallenden Spannungsimpulse überwacht.

6. Verfahren nach einem der Ansprüche 1 bis 5, **dadurch gekennzeichnet, dass** man die Leitfähigkeit der Mischung anhand des bei der Elektroporation durch die Mischung fließenden Stromes und/oder der an der Mischung abfallenden Spannung überwacht.

7. Verfahren nach einem der Ansprüche 1 bis 6, **dadurch gekennzeichnet, dass** man die Leitfähigkeit der Mischung vor der Elektroporation misst.

8. Verfahren nach einem der Ansprüche 1 bis 7, **dadurch gekennzeichnet, dass** der gesteuerte Parameter aus der nachstehenden Gruppe ausgewählt ist : Elektrische Feldstärke, die bei der Elektroporation aufrecht erhalten wird, Länge der Feldimpulse, die bei der Elektroporation verwendet werden, Abstand der Feldimpulse, die bei der Elektroporation verwendet werden, Gesamtanzahl der Feldimpulse, die bei der Elektroporation verwendet werden.

9. Verfahren nach einem der Ansprüche 1 bis 8, **dadurch gekennzeichnet, dass** der gesteuerte Parameter aus der nachstehenden Gruppe ausgewählt ist: Temperatur der Mischung bei Durchführung der Elektroporation, Druck, unter dem die Mischung bei der Elektroporation steht, Dauer der Elektroporationsbehandlung.

10. Verfahren nach einem der Ansprüche 1 bis 9, **dadurch gekennzeichnet, dass** ein gesteuerter Parameter das Verhältnis zwischen biologischem Material und Trägermaterial in der Mischung ist.

11. Verfahren nach einem der Ansprüche 1 bis 10, **dadurch gekennzeichnet, dass** einer der gesteuerten Parameter die Intensität einer Vorbehandlung, insbesondere eine Vorzerkleinerung des biologischen Materiales ist.

12. Verfahren nach Anspruch 11, wobei das biologische Material ein wenig oder keine Flüssigkeit enthaltendes biologisches Material ist, welchem in der Vorbehandlung eine Penetrationsflüssigkeit zugeführt wird, die den Zellen des biologischen Materiales eine schwache Leitfähigkeit gibt, **dadurch gekennzeichnet, dass** der gesteuerte Parameter ein Parameter der Vorbehandlung ist und aus der nachstehenden Gruppe ausgewählt ist: Die Behandlungszeit, die Art und Menge zugesetzter Penetrationsflüssigkeit, die Temperatur, bei welcher die Vorbehandlung ausgeführt wird, der Druck, bei welchem die Vorbehandlung ausgeführt wird, die Art und Intensität eines bei der Vorbehandlung erfolgenden Bewegens der Mischung aus biologischem Material und Penetrationsflüssigkeit.

13. Verfahren nach Anspruch 11 oder 12, **dadurch gekennzeichnet, dass** die Vorbehandlung eine mechanische Vorbehandlung, eine chemische Vorbehandlung mit einem Vorbehandlungsmittel oder eine physikalische Vorbehandlung umfasst, **dadurch gekennzeichnet, dass** der gesteuerte Parameter aus der nachstehenden Gruppe ausgewählt ist: Art und Konzentration des Vorbehandlungsmittels, Temperatur der Vorbehandlung, Intensität der Vorbehandlung, Bewegung des biologischen Materiales bei der Vorbehandlung.

14. Verfahren nach einem der Ansprüche 1 bis 13, bei welchem ein Materialstrom von einer Verfahrensstufe zu einer vorhergehenden Verfahrensstufe zurückgeführt wird, **dadurch gekennzeichnet, dass** der gesteuerte Parameter die Menge des zurückgeführten Materialstromes ist.

15. Verfahren nach Anspruch 14, wobei der zurückgeführte Materialstrom einer Zwischenbehandlung unterworfen wird, **dadurch gekennzeichnet, dass** der gesteuerte Parameter die Intensität der Zwischenbehandlung, die Dauer der Zwischenbehandlung, die Art oder Konzentration von bei der Zwischenbehandlung verwendeten Hilfsstoffen ist.

16. Verfahren nach einem der Ansprüche 1 bis 15, **dadurch gekennzeichnet, dass** bei Steuerung der Leitfähigkeit durch eine solche Parameteränderung, welche die Intensität der Elektroporationsbehandlung vermindert, die Fördergeschwindigkeit der Mischung vermindert wird und/oder der Abstand aufeinanderfolgender Feldimpulse verkleinert wird und/oder die Höhe und/oder Breite der Feldimpulse verkleinert wird.

17. Verfahren nach einem der Ansprüche 1 bis 15, **dadurch gekennzeichnet, dass** bei Steuerung der Leitfähigkeit durch eine Parameteränderung, welche die Intensität der Elektroporationsbehandlung vergrößert, die Fördergeschwindigkeit der Mischung erhöht wird und/oder der Abstand aufeinanderfolgender Feldimpulse vergrößert wird und/oder die Höhe und/oder Breite der Feldimpulse verkleinert wird.

18. Verfahren nach einem der Ansprüche 1 bis 17, **dadurch gekennzeichnet, dass** die Anzahl der Durchschläge,
die innerhalb eines vorgegebenen Zeitintervalles auftreten, gezählt werden und eine Notmaßnahme ergriffen wird,
wenn die festgestellte Anzahl von Durchschlägen größer ist als eine maximal zulässige Durchschlagszahl.

19. Verfahren nach Anspruch 18, **dadurch gekennzeichnet, dass** die Notmaßnahme aus folgender Gruppe ausgewählt ist: Abschalten des elektrischen Feldes für eine vorgegebene Zeitspanne oder bleibend, rasches Ausstoßen des Mischungsvolumens, welches gerade der Elektroporation unterworfen wird, aus dem Elektroporationsreaktor heraus, Fehlerhalt der Anlage.

20. Verfahren nach einem der Ansprüche 1 bis 19, wobei der Mischung zusätzlich ein die Leitfähigkeit beeinflussendes Additiv zugesetzt wird, **dadurch gekennzeichnet, dass** der gesteuerte Parameter die Menge des zugesetzten Additives ist.

21. Vorrichtung zur Durchführung des Verfahrens nach einem der Ansprüche 1 bis 20, mit einem Elektroporationsreaktor (20), der zwei einen Behandlungskanal (78) zumindest teilweise begrenzende Elektroden (80, 82) aufweist, die mit einem Hochspannungsimpulsgenerator (86, 90, 92, 94) verbunden sind, **dadurch gekennzeichnet, dass** eine Durchschlagsdetektionseinrichtung vorgesehen ist, welche eine oder mehrere Einrichtungen/Mittel aus der nachstehenden Gruppe umfasst: Ein Stromfühler (84) mit einem durch diesen beaufschlagten Amplitudendiskriminator oder Impulsbreitendiskriminator; eine Lichtdetektionseinrichtung, welche mit der Behandlungskammer (78) in optischer Verbindung steht; eine Druckfühleinrichtung, welche mit der Behandlungskammer (78) in Druckverbindung steht; ein Schallsensor, der akustisch mit dem Behandlungskanal (78) in Verbindung steht.

22. Vorrichtung nach Anspruch 21, **dadurch gekennzeichnet, dass** sie eine Leitfähigkeits-Messeinrichtung (84; 126; 128) aufweist, die mit der Mischung im Inneren des Behandlungskanales (78) zusammenarbeitet oder mit Mischung, die sich vor dem Behandlungskanal (78) befindet, zusammenarbeitet.

## Claims

1. Method for disintegrating biological material, in which
a) the biological material is dispersed in a carrier medium and
b) the mixture of carrier material and biological material is treated by electroporation,
**characterized in that**
c) the occurrence of electrical breakdowns is monitored during the electroporation treatment and
d) at least one parameter of the electroporation treatment is controlled in dependence of the result of the monitoring.

2. Method according to Claim 1, **characterized in that** one is monitoring the occurrence of breakdowns by means of light arising at the breakdowns.

3. Method according to Claim 1 or 2, **characterized in that** one is monitoring the occurrence of breakdowns by means of pressure surges arising at the breakdowns and/or by means of sound arising at the breakdowns.

4. Method according to one of Claims 1 to 3, **characterized in that** one is monitoring the breakdowns by means of the current pulses flowing through the mixture during electroporation and/or by means of the voltage pulses decaying at the mixture.

5. Method according to Claim 4, **characterized in that** one is monitoring the amplitude and/or the length of the current pulses flowing during electroporation and/or of the voltage pulses decaying at the mixture.

6. Method according to one of Claims 1 to 5, **characterized in that** one is monitoring the conductivity of the mixture by means of the current flowing through the mixture during electroporation and/or the voltage decaying at the mixture.

7. Method according to one of Claims 1 to 6, **characterized in that** one is measuring the conductivity of the mixture before electroporation.

8. Method according to one of Claims 1 to 7, **characterized in that** the controlled parameter is selected from the following group: electrical field strength maintained during electroporation, length of the field pulses used during electroporation, spacing of the field pulses used during electroporation, total number of field pulses used during electroporation.

9. Method according to one of Claims 1 to 8, **characterized in that** the controlled parameter is selected from the following group: temperature of the mixture when carrying out the electroporation, pressure to which the mixture is exposed during electroporation, duration of the electroporation treatment.

10. Method according to one of Claims 1 to 9, **characterized in that** one controlled parameter is the ratio between biological material and carrier material in the mixture.

11. Method according to one of Claims 1 to 10, **characterized in that** one of the controlled parameters is the intensity of a pretreatment, in particular a pre-comminution, of the biological material.

12. Method according to Claim 11, wherein the biological material is a biological material containing little or no liquid to which a penetration liquid is supplied in a pretreatment, which imparts weak conductivity to the cells of the biological material, **characterized in that** the controlled parameter is a pretreatment parameter and is selected from the following group: the treatment time, the type and quantity of penetration liquid added, the temperature at which the pretreatment is carried out, the pressure at which the pretreatment is carried out, the type and intensity of movement of the mixture of biological material and penetration liquid during pretreatment.

13. Method according to Claim 11 or 12, **characterized in that** the pretreatment includes a mechanical pretreatment, a chemical pretreatment with a pretreatment agent or a physical pretreatment, **characterized in that** the controlled parameter is selected from the following group: type and concentration of the pretreatment agent, temperature of the pretreatment, intensity of the pretreatment, movement of the biological material during pretreatment.

14. Method according to one of Claims 1 to 13, in which a material stream is recirculated from one method stage to a preceding method stage, **characterized in that** the controlled parameter is the quantity of the recirculated material stream.

15. Method according to Claim 14, wherein the recirculated material stream is subjected to an intermediate treatment, **characterized in that** the controlled parameter is the intensity of the intermediate treatment, the duration of the intermediate treatment, the type or concentration of auxiliary agents used during the intermediate treatment.

16. Method according to one of Claims 1 to 15, **characterized in that**, when controlling the conductivity by such a parameter change reducing the intensity of the electroporation treatment, the conveying speed of the mixture is reduced and/or the spacing between successive field pulses is shortened and/or the height and/or width of the field pulses is reduced.

17. Method according to one of Claims 1 to 15, **characterized in that**, when controlling the conductivity by a parameter change increasing the intensity of the electroporation treatment, the conveying speed of the mixture is increased and/or the spacing between successive field pulses is lengthened and/or the height and/or width of the field pulses is reduced.

18. Method according to one of Claims 1 to 17, **characterized in that** the number of breakdowns which occur within a predetermined time interval is counted and an emergency measure is taken when the determined number of breakdowns is greater than a maximum permissible number of breakdowns.

19. Method according to Claim 18, **characterized in that** the emergency measure is selected from the following group: switching off the electrical field for a predetermined time period or permanently, rapid ejection of the mixture volume which is currently being subjected to electroporation from the electroporation reactor, error stop of the installation.

20. Method according to one of Claims 1 to 19, wherein an additive which influences conductivity is additionally added to the mixture, **characterized in that** the controlled parameter is the quantity of the added additive.

21. Apparatus for carrying out the method according to one of Claims 1 to 20, having an electroporation reactor (20) which comprises two electrodes (80, 82) at least in part limiting a treatment channel (78), wherein the electrodes (80, 82) are connected to a high voltage pulse generator (86, 90, 92, 94), **characterized in that** a breakdown detection device is provided which includes one or more devices/means from the following group: a current sensor (84) with an amplitude discriminator or pulse width discriminator acted upon by said current sensor (84); a light detection device which is in optical connection with the treatment chamber (78); a pressure sensing device which is in pressure connection with the treatment chamber (78); an sound sensor which is in acoustic connection with the treatment channel (78).

22. Apparatus according to Claim 21, **characterized in that** it comprises a conductivity measurement device (84; 126; 128) which cooperates with the mixture in the interior of the treatment channel (78) or with the mixture located upstream of the treatment channel (78).

## Revendications

1. Procédé de solubilisation d'une matière biologique, selon lequel
a) la matière biologique est répartie dans un milieu porteur et
b) le mélange de matière porteuse et de matière biologique est traité par électroporation,
**caractérisé en ce que**
c) l'apparition de claquages électriques pendant le traitement par électroporation est surveillée et
d) au moins un paramètre du traitement par électroporation est commandé en fonction du résultat de la surveillance.

2. Procédé selon la revendication 1, **caractérisé en ce que** l'on surveille l'apparition de claquages sur la base de la lumière produite lors des claquages.

3. Procédé selon la revendication 1 ou 2, **caractérisé en ce que** l'on surveille l'apparition de claquages sur la base de chocs de pression produits lors des claquages et/ou du bruit produit lors des claquages.

4. Procédé selon l'une des revendications 1 à 3, **caractérisé en ce que** l'on surveille les claquages sur la base des impulsions de courant circulant à travers le mélange et/ou sur la base des impulsions de tension aux bornes du mélange lors de l'électroporation.

5. Procédé selon la revendication 4, **caractérisé en ce que** l'on surveille l'amplitude et/ou la longueur des impulsions de courant circulant et/ou des impulsions de tension aux bornes du mélange lors de l'électroporation.

6. Procédé selon l'une des revendications 1 à 5, **caractérisé en ce que** l'on surveille la conductivité du mélange sur la base du courant circulant à travers le mélange et/ou de la tension aux bornes du mélange lors de l'électroporation.

7. Procédé selon l'une des revendications 1 à 6, **caractérisé en ce que** l'on mesure la conductivité du mélange avant l'électroporation.

8. Procédé selon l'une des revendications 1 à 7, **caractérisé en ce que** le paramètre commandé est choisi dans le groupe suivant : intensité de champ électrique maintenue lors de l'électroporation, longueur des impulsions de champ utilisées lors de l'électroporation, espacement des impulsions de champ utilisées lors de l'électroporation, nombre total des impulsions de champ utilisées lors de l'électroporation.

9. Procédé selon l'une des revendications 1 à 8, **caractérisé en ce que** le paramètre commandé est choisi dans le groupe suivant : température du mélange lors de l'électroporation, pression à laquelle le mélange est soumis lors de l'électroporation, durée du traitement par électroporation.

10. Procédé selon l'une des revendications 1 à 9, **caractérisé en ce qu'**un paramètre commandé est le rapport entre matière biologique et matière porteuse dans le mélange.

11. Procédé selon l'une des revendications 1 à 10, **caractérisé en ce que** l'un des paramètres commandés est l'intensité d'un prétraitement, en particulier un prébroyage de la matière biologique.

12. Procédé selon la revendication 11, dans lequel la matière biologique est une matière biologique contenant peu ou pas de liquide, auquel un liquide de pénétration est fourni lors du prétraitement, lequel confère une faible conductivité aux cellules de la matière biologique, **caractérisé en ce que** le paramètre commandé est un paramètre du prétraitement et est choisi dans le groupe suivant : le temps de traitement, le type et la quantité de liquide de pénétration ajouté, la température à laquelle le prétraitement est effectué, la pression à laquelle le prétraitement est effectué, le type et l'intensité d'un mouvement du mélange de matière biologique et de liquide de pénétration ayant lieu lors du prétraitement.

13. Procédé selon la revendication 11 ou 12, **caractérisé en ce que** le prétraitement comprend un prétraitement mécanique, un prétraitement chimique avec un agent de prétraitement ou un prétraitement physique, **caractérisé en ce que** le paramètre commandé est choisi dans le groupe suivant : type et concentration de l'agent de prétraitement, température du prétraitement, intensité du prétraitement, mouvement de la matière biologique lors du prétraitement.

14. Procédé selon l'une des revendications 1 à 13, dans lequel un courant de matière est renvoyé d'une étape de procédé à une étape de procédé précédente, **caractérisé en ce que** le paramètre commandé est la quantité du courant de matière renvoyé.

15. Procédé selon la revendication 14, dans lequel le courant de matière renvoyé est soumis à un traitement intermédiaire, **caractérisé en ce que** le paramètre commandé est l'intensité du traitement intermédiaire, la durée du traitement intermédiaire, le type ou la concentration de substances auxiliaires utilisées lors du traitement intermédiaire.

16. Procédé selon l'une des revendications 1 à 15, **caractérisé en ce que**, lorsque la conductivité est commandée par un changement de paramètre qui diminue l'intensité du traitement par électroporation, la vitesse de transport du mélange est diminuée et/ou l'espacement d'impulsions de champ successives est réduit et/ou la hauteur et/ou la largeur des impulsions de champ est réduite.

17. Procédé selon l'une des revendications 1 à 15, **caractérisé en ce que**, lorsque la conductivité est commandée par un changement de paramètre qui augmente l'intensité du traitement par électroporation, la vitesse de transport du mélange est augmentée et/ou l'espacement d'impulsions de champ successives est augmenté et/ou la hauteur et/ou la largeur des impulsions de champ est réduite.

18. Procédé selon l'une des revendications 1 à 17, **caractérisé en ce que** le nombre de claquages qui apparaissent dans un intervalle de temps prédéfini est compté et une mesure d'urgence est prise si le nombre déterminé de claquages est supérieur à un nombre maximal admissible de claquages.

19. Procédé selon la revendication 18, **caractérisé en ce que** la mesure d'urgence est choisie dans le groupe suivant : coupure du champ électrique pendant une durée prédéfinie ou de façon permanente, expulsion rapide du volume de mélange actuellement soumis à l'électroporation du réacteur d'électroporation, arrêt sur erreur de l'installation.

20. Procédé selon l'une des revendications 1 à 19, selon lequel un additif influençant la conductivité est ajouté en plus au mélange, **caractérisé en ce que** le paramètre commandé est la quantité de l'additif ajouté.

21. Dispositif pour mettre en oeuvre le procédé selon l'une des revendications 1 à 20, comprenant un réacteur d'électroporation (20) présentant deux électrodes (80, 82) délimitant au moins partiellement un canal de traitement (78) et reliées à un générateur d'impulsions haute tension (86, 90, 92, 94), **caractérisé en ce qu'**un moyen de détection de claquage est prévu, lequel comprend un ou plusieurs équipements/moyens choisis dans le groupe suivant : un capteur de courant (84) avec un discriminateur d'amplitude ou un discriminateur de largeur d'impulsion sollicité par celui-ci ; un moyen de détection de lumière en communication optique avec la chambre de traitement (78) ; un moyen de détection de pression en communication de pression avec la chambre de traitement (78) ; un capteur acoustique en communication acoustique avec le canal de traitement (78).

22. Dispositif selon la revendication 21, **caractérisé en ce qu'**il présente un moyen de mesure de conductivité (84; 126; 128) qui coopère avec le mélange à l'intérieur du canal de traitement (78) ou avec le mélange qui se trouve avant le canal de traitement (78).
